# EUROPEAN PATENT SPECIFICATION

(11) **EP 2 305 685 B1**
(45) Date of publication and mention of the grant of the patent: **21.05.2014**
(21) Application number: 09012372.0
(22) Date of filing: 30.09.2009
(51) Int. Cl.: C07D 495/04, H01B 1/12, H01G 9/025, C08K 5/13, C08K 5/17, C08K 5/132, C08K 5/134, C08K 5/3435, C08K 5/3492, C08L 65/00

(54) **Stabilised thiophene derivatives**
Stabilisierte Thiophen-Derivate
Dérivés stabilisés à base de thiophène

(43) Date of publication of application: 06.04.2011
(73) Proprietor: Heraeus Precious Metals GmbH & Co. KG, 63450 Hanau (DE)
(72) Inventor: Reuter, Knud Dr., 47800 Krefeld (DE); Wussow, Klaus Dr., 57250 Netphen (DE); Merker, Udo Dr., 51105 Köln (DE); Elschner, Andreas Dr., 45468 Mülheim/ Ruhr (DE)
(74) Representative: Herzog, Martin

(56) References cited:
- EP-A- 1 154 449
- EP-A- 1 860 111
- WO-A-2008/055834
- BRUNO FABRE, PHILIPPE MARREC, JACQUES SIMONET: "Electroactive Materials Containing Macrocyclic Pseudo-Crown Ether Cavities Electroformed from a Solid-State Electropolymerization Reaction" JOURNAL OF THE ELECTROCHEMICAL SOCIETY, vol. 145, no. 12, December 1998 (1998-12), pages 4110-4119, XP002567301 Electrochem. Soc., USA
- DATABASE CA [Online] CHEMICAL ABSTRACTS SERVICE, COLUMBUS, OHIO, US; 2 March 2006 (2006-03-02), HUANG, WEI ET AL: "Conjugate luminescent polymer having antioxidant group in side chain, and its application" XP002567302 retrieved from STN Database accession no. 2006:187210 -& CN 1 673 310 A (FUDAN UNIVERSITY, PEOP. REP. CHINA) 28 September 2005 (2005-09-28)

## Description

The invention relates to the use of a stabiliser.

Solid electrolytic capacitors with conductive polymers as the cathode materials have been widely used in the electronics industry due to their advantageously low equivalent series resistance (ESR) and *"non-burning*/*non-ignition"* failure mode. Various types of conductive polymers including polypyrrole, polyaniline, and poly(3,4-ethyldioxythiophene) (PEDOT) are applied to electrolytic capacitors as a cathode material when valve metals such as Ta, Al, and Nb as well as conductive oxides such as ceramic NbO, are used as the anode. In a manufacturing process to produce conductive polymer based valve metal capacitors, Ta powder, for example, is mechanically pressed to form Ta metal pellets, which are subsequently sintered at high temperature under vacuum. The sintered pellets are anodized in an electrolyte solution to form a dielectric layer (Ta₂O₅) on the anode surface. Following that, multiple layers of a conductive polymer, such as poly 3,4-ethylenedioxythiophene (PEDOT), are laid down by a multiple dipping polymerization process. During the polymerization process, an oxidant solution, such as iron(III) p-toluenensulfonate solution in a solvent, is in some cases first applied onto the anodes. It is then followed by the application of a liquid monomer or monomer solution as disclosed by D Wheeler, et al in U.S. Patent No. 6,136,176 and by R. Hahn, et al., in U.S. Patent 6,334,966.

For the purpose of achieving high electrical conductivities in this connection, the purity of the thiophene derivatives monomers used for the preparation of the conductive polymer plays a crucial role. Ordinarily, distillation is used as a conventional purification process for thiophene derivatives.

As disclosed in EP 1 860 111 Al thiophenes that have been purified in this way have a tendency in the course of storage towards changes in colour and/or the formation of undesirable secondary components, such as, for example, the formation of dimeric or trimeric thiophenes having the structures shown below. This results in considerable impairment of the properties of the polythiophenes prepared therefrom.

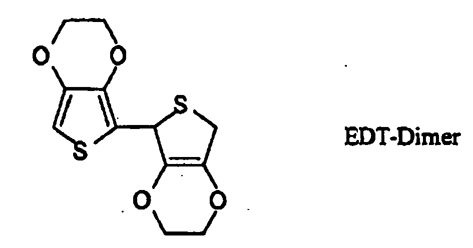

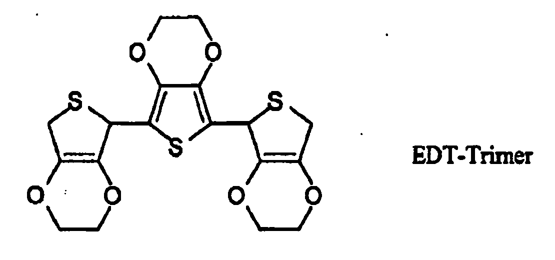

In order to avoid these negative effects of non-conjugated dimeric or trimeric thiophene derivatives, EP 1860 111 Al suggests to pre-treat the thiophene derivatives with alkaline materials which, before the monomer is used for forming a capacitor, is separated from the monomer, for example by filtration. Alternatively, both documents suggest passing the monomer solution through a basic anion exchange column.

Although by the method disclosed in EP 1 860 111 A1 the storage time of thiophenes can be significantly improved, the disadvantage of this method can be seen in the fact that, whenever the monomer is used, an extra step of pre-treating the monomer with the basic material has to be performed.

An object of the present invention is thus to reduce or even overcome the disadvantages of the state of the art.

In particular it is an object of the present invention to provide a stabilised monomer composition of 3,4-ethylenedioxythiophene that does not have to be pre-treated with an alkaline material, which subsequently has to be removed before the composition is used for the manufacture of an electronic capacitor.

It was furthermore an object of the present invention to provide a process for the manufacture of an electronic capacitor with reproducible low ESR-values which, compared to the processes disclosed in the prior art, requires fewer process steps.

A contribution to the solution of at least one of the above objects is provided by the subject matter of the category-forming independent principal claim, whereby the therefrom dependent sub-claims represent preferred embodiments of the present invention, whose subject matters likewise make a contribution to solving at least one object.

The invention relates to the use of a stabiliser as defined in claim 1.

The stabilised monomer composition comprises:
- at least 90 wt.-%, preferably at least 95 wt.-%, more preferably at least 97,5 wt.-% and most preferably at least 98,5 wt.-%, in each case based on the total weight of the stabilised monomer, 3,4-ethylenedioxythiophene as the monomer,
   and
- 0.001 to 10 wt.-%, preferably 0.01 to 5 wt.-%, more preferably 0.05 to 2.5 wt.-% and most preferably 0.1 to 1.5 wt-%, in each case based on the total weight of the stabilised monomer, of a stabiliser, which is preferably a radical scavenger, an amine or an UV/VIS-absorber.

Surprisingly, it has now been found that it is possible to stabilise 3,4-ethylenedioxythiophene by adding stabilisers such as radical scavengers, amines or UV/VIS-absorbers, which do not have to be removed prior to the use of the 3,4-ethylenedioxythiophene for forming electronic capacitors. The person skilled in the art could not expect that the ESR of capacitors that have been formed by polymerizing 3,4-ethylenedioxythiophene in the presence of such stabilisers can be kept low in a reproducible manner. In this context it was particularly surprising that the formation of dimeric and trimeric thiophenes can be inhibited by the addition of stabilisers such as radical scavengers and UV/VIS-absorbers as it was known that the formation of these compounds is an acid catalyzed, ionic reaction.

The stabilised monomer composition comprises, besides 3,4-ethylenedioxythiophene, a stabiliser. As a *"stabiliser"* in the sense of the present invention it is understood a compound that inhibits the formation of dimeric or trimeric thiophene derivatives in the monomer during storage of the monomer, especially when the monomer is exposed to UV/VIS-light.

According to a first preferred embodiment of the stabilised monomer composition the stabiliser may be a radical scavenger.

As a *"radical scavenger"* in the sense of the present invention it is understood a compound that, when reacting with a radical, forms a reaction product that is less reactive than the radical with which the compound has reacted.

According to a preferred embodiment of the stabilised monomer composition in which the stabiliser is a radical scavenger, the stabiliser may be a so called *"HALS"* ("*Hindered amine light stabilizer*")*.*

In this context, the radical scavenger may have a chemical structure comprising at least one structural element having the general formula (III) 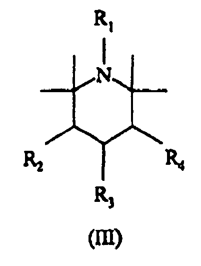 in which
R₁ stands for a hydrogen atom, a hydroxyl group, a linear or branched C₁-C₁₈-alkyl residue or a linear or branched C₁-C₁₈-alkoxy residue, wherein a hydrogen atom, a methyl group or a H₁₇C₈-O-group are especially preferred as residues a R₁, and
R₂, R₃ and R₄ stand, independently of one another, for a hydrogen atom or an organic residue.

In context with this preferred embodiment of the stabilised monomer composition, the radical scavenger may have a chemical structure comprising of the general formula (IV) 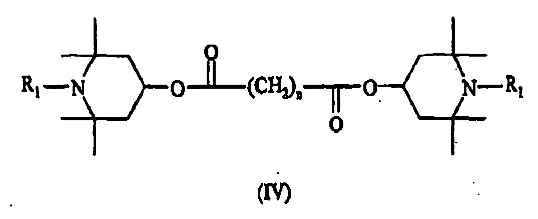 in which R₁ is a residue having the significance stated before and n is an integer ranging from 2 to 20, wherein a hydrogen atom, a methyl group or a H₁₇C₈-O-group are especially preferred as residues a R₁ and wherein the value of n is preferably within the range from 6 to 10, a value for n of 8 being most preferred. Examples of appropriate radical scavengers that can be mentioned in this context are the products Tinuvin^{®}123, Tinuvin^{®}292, Tinuvin^{®}765 and Tinuvin^{®}770 DF which can be obtained from Ciba AG, Basel, Switzerland.

In context with this preferred embodiment of the stabilised monomer composition, the radical scavenger may also have a chemical structure comprising of the general formula (V) 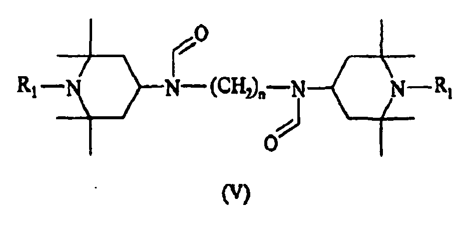 in which R₁ is a residue have the significance stated in claim 5 and n is an integer ranging from 2 to 20, wherein a hydrogen atom, a hydroxyl group, a methyl group or a H₁₇C₈-O-group are especially preferred as residues a R₁ and wherein the value of n is preferably within the range from 4 to 8, a value for n of 6 being most preferred. An example of an appropriate radical scavenger that can be mentioned in this context is the product Uvinul^{®}4050 H from BASF AG, Ludwigshafen, Germany.

According to another preferred embodiment of the stabilised monomer composition, in which the stabiliser is a radical scavenger, the stabiliser may be a phenol derivative, for example a sterically hindered phenol derivative, a brenzcatechin derivative or a hydrochinone derivative.

In context with this preferred embodiment of the stabilised monomer composition, the radical scavenger may have a chemical structure comprising at least one structural element having the general formula (VI) 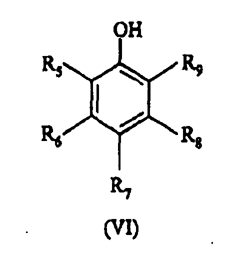 in which R₅, R₆, R₇, R₈ and R₉ stand, independently of one another, for a hydrogen atom, a hydroxyl group or an organic residue, with the provisio that at least one of the residues R₅, R₆, R₇, R₈ and R₉, preferably at least the residue adjacent to the OH-group, is a -C(CH₃) residue. Examples of appropriate radical scavengers that can be mentioned in this connection are 2,2'-methylen-bis-(6-tert-butyl-4-ethyl)-phenol, tert-butylhydroxy anisol, tert-butylbrenzcatechin and 4-tert-butylcatechol. In this context it may furthermore be preferred that at least one of the residues R₅ and R₇ is a hydroxyl group.

In context with this preferred embodiment of the stabilised monomer composition , the radical scavenger may also have a chemical structure comprising at least one structural element having the general formula (VII) 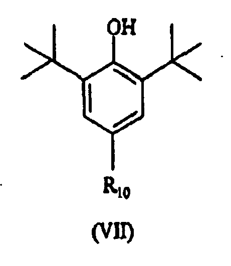 in which R₁₀ stands for a hydrogen atom, a hydroxyl group or an organic residue, wherein it is especially preferred that residue R₁₀ also comprises a structural element having the general formula (VII). Examples of appropriate radical scavengers that can be mentioned in this context are the products Irganox^{®}1076, Irganox^{®}MD 1024 and Irganox^{®}1010, 076 from Ciba AG, Basel, Switzerland, and Tinuvin^{®}1130 from BASF AG, Ludwigshafen, Germany.

In context with this preferred embodiment of the stabilised monomer composition, the radical scavenger may furthermore have a chemical structure comprising at least one structural element having the general formula (VIII) 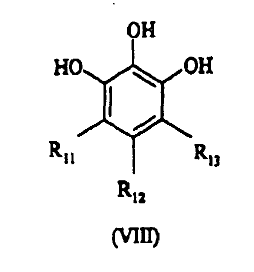 in which R₁₁, R₁₂ and R₁₃ stand, independently of one another, for a hydrogen atom or an organic residue, wherein a hydrogen atom and a C₁-C₁₀-alkyl residue are especially preferred. Examples of appropriate radical scavengers that can be mentioned in this context are propyl gallate and pyrogallol.

According to a second preferred embodiment of the stabilised monomer composition the stabiliser may be an amine.

According to a preferred embodiment of the stabilised monomer composition , in which the stabiliser is an amine, the stabiliser may have a chemical structure having the general formula (IX) 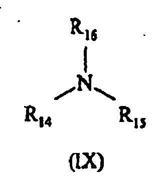 in which R₁₄, R₁₅ and R₁₆ stand, independently of one another, for a hydrogen atom, C₁-C₂₀-alkyl residue or an C₁-C₅-hydroxyalkl residue, wherein it may be advantageous, that at least one of the residues R₁₄, R₁₅ and R₁₆ is an C₁-C₅-hydroxyalkyl residue. Examples of appropriate radical scavengers that can be mentioned in this context are N-methyldiethanolamine, 2-dimethylaminoethanaol and triethanolamine.

According to a third preferred embodiment of the stabilised monomer composition the stabiliser may be an UV/VIS-absorbent, preferably an UV-absoxbent. Examples of appropriate UV/VIS-absorbents that can be mentioned in this context are the products Tinuvin^{®}1130 from Ciba AG, Basel, Switzerland, and Uvinul^{®}DS 49 from BASF AG, Ludwigshafen, Germany.

In accordance with the present invention the stabiliser is at least partially soluble in 3,4-ethylenedioxythiophene. A stabiliser is understood to be at least partially soluble in 3,4-ethylenedioxythiophene if the stabiliser, when combined with the monomer in the relative amount as indicated above, can be completely dissolved in the monomer without the formation of a separate liquid phase or a remaining solid mass.

3,4-ethylenedioxythiophene that has been stabilised according to the present invention tend distinctly less towards changes in the formation of secondary components, such as dimeric or trimeric thiophenes, and therefore has a distinctly higher stability in storage. In its properties it differs significantly from 3,4-ethylenedioxythiophene that has not been stabilised in accordance with the present the invention.

The stabilised monomer composition is contained in a closed container. As containers, bottles, vessels and barrels a preferred. The inner volume of these containers is preferably at least 50 ml, more preferably at least 100 ml and most preferably at least 1000 ml. It is furthermore preferred that the bottle can be closed by means of a screw top. Moreover, the container can be a glass container, a plastic container or a metal container. Furthermore, the container is preferably impermeable for light, preferably for UV-light.

The stabilised monomer composition can be used in a method for the manufacture of a capacitor, preferably an electronic capacitor, comprising
- forming an anode of a valve metal;
- forming a precursor comprising said anode and a dielectric;
- adding an intrinsically conductive polymer on said precursor, wherein said intrinsically conductive polymer is based on the stabilised monomer .

In addition to the dielectric and the anode, the precursor may comprise one or more further layers. According to one embodiment of the present invention, application of the stabilised monomer to the dielectric of the anode can be effected directly or using an adhesion promoter, for example a silane, for example organofunctional silanes or hydrolysates thereof, e.g. 3-glycidoxypropyltrialkoxysilane, 3-aminopropyltriethoxysilane, 3-mercaptopropyltrimethoxysilane, 3-methacryloyloxypropyltrimethoxysilane, vinyltrimethoxysilane or octyltriethoxysilane, and/or one or more other functional layers. The intrinsically conductive polymer is preferably than based on the stabilised monomer if the polymer is formed by polymerizing it from the stabilised monomers.

In a first step an anode is formed of a valve metal, wherein a valve metal powder with a high surface area is compressed and sintered to a porous electrode body. When this is done, an electrical contact wire, preferably of a valve metal, for example tantalum, is typically also compressed into the electrode body. It is alternatively also possible to etch metal foils in order to obtain a porous film. In the case of a wound capacitor, a porous anode film which forms the electrode body and a cathode film are separated by a separator and wound up.

Valve metals are understood within the scope of the invention to denote those metals whose oxide layers do not permit current to flow equally in both directions. When a voltage is applied to the anode the oxide layers of the valve metals prevent the flow of current, whereas when a voltage is applied to the cathode large currents flow that can destroy the oxide layer. Valve metals include Be, Mg, Al, Ge, Si, Sn, Sb, Bi, Ti, Zr, Hf, V, Nb, Ta and W as well as an alloy or compound of at least one of these metals with other elements. The best known examples of valve metals are Al, Ta and Nb. Compounds with comparable properties are those exhibiting metallic conductivity that can be oxidised and whose oxide layers have the properties described hereinbefore. For example NbO exhibits metallic conductivity, but is generally not regarded as a valve metal. Layers of oxidised NbO however exhibit the typical properties of valve metal oxide layers, so that NbO or an alloy or compound of NbO with other elements are typical examples of such compounds with comparable properties.

Consequently the term "*oxidisable metal*" covers not only metals but also an alloy or compound of a metal with other elements, provided that they exhibit metallic conductivity and can be oxidised.

Particularly preferably the valve metal or the compound with comparable properties is tantalum, niobium, aluminium, titanium, zirconium, hafnium, vanadium, an alloy or compound of at least one of these metals with other elements, NbO or an alloy or compound of NbO with other elements.

In a second step, a precursor comprising said anode and a dielectric is formed.

The dielectric is preferably formed on said anode. The dielectric preferably consists of an oxide of the electrode material or - in the case where this is already an oxide - of a higher oxidised form of the electrode material. The dielectric optionally contains further elements and/or compounds. The oxidisable metals are for example sintered in powder form to form a porous electrode body or a porous structure is impressed on a metallic body. The latter procedure may be carried out for example by etching a film. The porous electrode bodies are for example oxidised in a suitable electrolyte, such as for example phosphoric acid, by applying a voltage such that such a so called *"oxidised electrode body*" is obtained. The magnitude of this forming voltage depends on the oxide layer thickness to be achieved or on the subsequent application voltage of the capacitor. Preferred voltages are 1 to 300 V, particularly preferably 1 to 80 V.

In a third step of one embodiment of the method for the manufacture of a capacitor an intrinsically conductive polymer is added on said precursor, wherein said intrinsically conductive polymer is based on the stabilised monomer composition. In this context it is preferred that said precursor, preferably the dielectric of the precursor, is coated with a solution of the stabilised monomer composition and that subsequently said monomer is polymerised, preferably by means of oxidative or electrochemical polymerization, especially preferred by means of oxidative polymerization using appropriate oxidising agents, such that the intrinsically conductive polymer is obtained.

Optionally further layers, for example polymeric outer layer are applied. A coating containing good conducting layers, such as graphite and silver, or a metallic cathode body, serves as electrode for the discharge of the current. Finally the capacitor is contacted and encapsulated.

As oxidising agents there may be used all suitable metal salts known to the person skilled in the art for the oxidative polymerisation of thiophenes.

Metal salts suitable as oxidising agent are metal salts of main group metals or subgroup metals, the latter hereinafter also being termed transition metal salts, of the Periodic System of the Elements. Suitable transition metal salts are in particular salts of an inorganic or organic acid or of an inorganic acid comprising organic radicals, of transition metals such as for example iron(III), copper(II), chromium(VI), cerium(IV), manganese(IV) and manganese(VII) and ruthenium(III).

Preferred transition metal salts are those of iron(III). Conventional iron(III) salts are advantageously inexpensive, readily obtainable and are easy to handle, such as for example iron(III) salts of inorganic acids, such as for example iron(III) halides (e.g. FeCl₃) or iron(III) salts of other inorganic acids, such as Fe(ClO₄)₃ or Fe₂(SO₄)₃, and iron(III) salts of organic acids and of inorganic acids comprising organic radicals.

As iron(III) salts of inorganic acids comprising organic radicals, there may for example be mentioned iron(III) salts of sulfuric acid monoesters of C₁-C₂₀-alkanols, e.g. the iron(III) salt of lauryl sulfate.

Particularly preferred transition metal salts are those of an organic acid, in particular iron(III) salts of organic acids.

As iron(III) salts of organic acids, the following may for example be mentioned: iron(III) salts of C₁-C₂₀-alkanesulfonic acids, such as methanesulfonic, ethanesulfonic, propanesulfonic, butanesulfonic or higher sulfonic acids such as dodecanesulfonic acid, of aliphatic perfluorosulfonic acids, such as trifluoromethanesulfonic acid, perfluorobutanesulfonic acid or perfluorooctanesulfonic acid, of aliphatic C₁-C₂₀-carboxylic acids such as 2-ethylhexylcarboxylic acid, of aliphatic perfluorocarboxylic acids such as trifluoroacetic acid or perfluorooctanoic acid, and of aromatic sulfonic acids optionally substituted by C₁-C₂₀-alkyl groups, such as benzenesulfonic acid, o-toluenesulfbnic acid, p-toluenesulfonic acid or dodecylbenzenesulfonic acid, and of cycloalkanesulfonic acids such as camphorsulfonic acid.

Arbitrary mixtures of these aforementioned iron(III) salts of organic acids may also be used as oxidising agent.

The use of iron(III) salts of organic acids and of inorganic acids comprising organic radicals has the great advantage that they do not have a corrosive effect.

Most particularly preferred as metal salts are iron(III) p-toluenesulfonate, iron(III) o-toluenesulfonate or a mixture of iron(III) p-toluenesulfonate and iron(III) o-toluenesulfonate.

Further suitable oxidising agents are peroxo compounds such as peroxodisulfates (persulfates), in particular ammonium and alkali metal peroxodisulfates, such as sodium and potassium peroxodisulfate, or alkali metal perborates - optionally in the presence of catalytic amounts of metal ions such as iron, cobalt, nickel, molybdenum or vanadium ions - as well as transition metal oxides, such as for example manganese dioxide (manganese(IV) oxide) or cerium(IV) oxide.

For the oxidative polymerisation of thiophenes, there are theoretically required 2.25 equivalents of oxidising agent per mole of thiophene (see for example J. Polym. Sc. Part A Polymer Chemistry Vol. 26, p. 1287 (1988)). Smaller or larger amounts of equivalents of oxidising agent may however also be used.

The conducting polymers contained as solid electrolyte in the capacitors produced by the method according to the invention are cationic. In order to compensate the positive charge the cationic conducting polymers require anions as counter-ions.

Counter-ions may be monomeric or polymeric anions, the latter hereinafter being termed polyanions. Polymeric anions may for example be anions of polymeric carboxylic acids, such as polyacrylic acids, polymethacrylic acid or polymaleic acids, or polymeric sulfonic acids, such as polystyrenesulfonic acids and polyvinylsulfonic acids. These polycarboxylic and polysulfonic acids may also be copolymers of vinylcarboxylic acids and vinylsulfonic acids with other polymerisable monomers, such as acrylic acid esters and styrene.

Monomeric anions are preferably used for the solid electrolytes since they more readily penetrate the oxidised electrode body.

The following may for example serve as monomeric anions: monomeric anions of C₁-C₂₀-alkanesulfonic acids, such as methanesulfonic, ethanesulfonic, propanesulfonic, butanesulfonic or higher sulfonic acids such as dodecanesulfonic acid, of aliphatic perfluorosulfonic acids, such as trifluoromethanesulfonic acid, perfluorobutanesulfonic acid or perfluorooctanesulfonic acid, of aliphatic C₁-C₂₀-carboxylic acids such as 2-ethylhexylcarboxylic acid, of aliphatic perfluorocarboxylic acids such as trifluoroacetic acid or perfluorooctanoic acid, and of aromatic sulfonic acids optionally substituted by C₁-C₂₀-alkyl groups, such as benzenesulfonic acid, o-toluenesulfonic acid, p-toluenesulfonic acid or dodecylbenzenesulfonic acid, and of cycloalkanesulfonic acids such as camphorsulfonic acid, or tetrafluoroborates, hexafluorophosphates, perchlorates, hexafluoroantimonates, hexafluoroarsenates or hexachloroantimonates. The monomeric anions of sulfonic acids are not restricted to those of monosulfonic acids, but may also be anions of disulfonic, trisulfonic or polysulfonic acids, for example of benzenedisulfonic acid or naphthalenedisulfonic acid.

The anions of p-toluenesulfonic acid, methanesulfonic acid or camphorsulfonic acid are preferred.

The counter-anions are added preferably to the oxidizer or the stabilised monomer in the form of their alkali metal salts or as free acids.

The possibly present anions of the oxidising agent that is used may also serve as counter-ions, which means that it is not absolutely essential to add additional counter-ions.

As stated above, the precursor is coated with a solution of the stabilised monomer composition, which in some cases can be provided in a solution and in other cases as such. The following organic solvents that are inert under the reaction conditions may in particular be mentioned as solvents for the stabilised monomer for the production of conducting polymers and/or oxidising agents and/or counter-ions: aliphatic alcohols such as methanol, ethanol, i-propanol and butanol; aliphatic ketones such as acetone and methyl ethyl ketone; aliphatic carboxylic acid esters such as ethyl acetate and butyl acetate; aromatic hydrocarbons such as toluene and xylene; aliphatic hydrocarbons such as hexane, heptane and cyclohexane; chlorinated hydrocarbons such as dichloromethane and dichloroethane; aliphatic nitriles such as acetonitrile; aliphatic sulfoxides and sulfones such as dimethyl sulfoxide and sulfolane; aliphatic carboxylic acid amides such as methylacetamide, dimethylacetamide and dimethylformamide; aliphatic and araliphatic ethers such as diethyl ether and anisole. In addition water or a mixture of water with the aforementioned organic solvents may also be employed as solvent.

In the method for the manufacture of a capacitor it is further preferred that said adding comprises
- coating said precursor with a solution of the stabilised monomer composition;
- polymerizing said monomer such that the intrinsically conductive polymer is obtained.

The intrinsically conductive polymer to the solid electrolyte can be added to the precursor by polymerisation of the stabilised monomer composition with oxidising agents or by electrochemical polymerisation of the monomer on the precursor. For the polymerisation of the monomer with oxidising agents, monomer and oxidizing agents can be applied as a mixture or successively to the electrode body covered by a dielectric.

The oxidising agents and the stabilised monomer composition may be mixed together in solid and/or liquid form and then applied to the oxidised electrode body anode to from the solid electrolyte layer. One or more solvents is/are however preferably added to the mixtures. As suitable solvents there may be mentioned in particular the solvents already listed above.

Oxidising agents and the stabilised monomer composition can also be applied successively, in some cases in the form of solutions, to the oxidised electrode body for forming the solid electrolyte layer. When the monomer is applied first, it is preferred that the monomer is diluted with a solvent. As suitable solvents there may be mentioned in particular the solvents already listed above.

The stabilised monomer composition can also be electrochemically polymerized in the oxidised electrode body for forming the solid electrolyte layer.

Oxidative polymerisation is carried out at temperatures from -10° to 300°C, preferably 10° to 200°C, particularly preferably 30° to 150°C. The duration of the heat treatment depends on the nature of the polymer used for the coating and ranges from 5 seconds up to several hours. Temperature profiles with different temperatures and residence times may also be used for the thermal treatment.

It may be advantageous to wash out the excess oxidising agent and residual salts from the coating using a suitable solvent, preferably water or alcohols. Residual salts are understood in this connection to mean the reduced forms of the oxidising agent and possibly further salts that may be present.

For metal oxide dielectrics, such as for example oxides of valve metals, it may be advantageous to treat the oxide film electrochemically in order to rectify possible defects in the said oxide film and thereby reduce the residual current of the finished capacitor. In this so-called reforming the capacitor body is immersed in an electrolyte and a positive voltage is applied with positive potential to the electrode body. The electric current, which flows from the electrode body over defective sites in the oxide film to the conductive polymer, reforms the oxide film at these sites or destroys the conductivity of the conductive polymer at these defective sites.

Depending on the nature of the oxidised electrode body it may be advantageous to impregnate the oxidised electrode body before and/or after a wash procedure either one, two or several further times with the mixtures of the stabilised monomer composition and oxidising agents or successively with the monomer and oxidising agents in order to achieve thicker polymer layers in the interior of the electrode body. The compositions of the mixtures may in this connection vary. The solid electrolyte may optionally be composed of a multilayer system that comprises a plurality of functional layers.

According to a preferred embodiment of the method for the manufacture of a capacitor it is preferred that the polymerization of the 3,4-ethylenedioxythiophene takes place in the presence of at least a part of the stabiliser. It is furthermore preferred that the monomer has not been pre-treated with an alkaline material, which, after the pre-treatment, has at least partially been removed from the monomer, before the monomer is applied in the form of a solution on the dielectric.

The capacitors manufactured in accordance with the method are outstandingly suitable, owing to their low ESR, for use as a component in electronic circuits, for example as filter capacitors or decoupling capacitors. The use also forms part of the subject-matter of the invention. Preference is given to electronic circuits, as present, for example, in computers (desktops, laptops, servers), in computer peripherals (e.g. PC cards), in portable electronic devices, for example mobile phones, digital cameras or amusement electronics, in devices for amusement electronics, for example in CD/DVD players and computer game consoles, in navigation systems, in telecommunications equipment, in domestic appliances, in voltage supplies or in automotive electronics.

The following Examples serve for exemplary elucidation of the invention and are not to be interpreted as a restriction.

### EXAMPLES

### Examples 1-15:

5 g pure 3,4-ethylendioxythiophen ("EDOT"; Clevios M V2; H.C. Starck Clevios GmbH) were mixed with 1.0 % by weight (50 mg) of different stabilisers by stirring at 23°C in an open beaker. 1 ml of each mixture in a glass beaker was irradiated for 6 hours with UV light, wavelength λ = 366 nm, by a high pressure Hg-lamp (DESAGA HP-UVIS; Desaga GmbH, Wiesloch, Germany). The open samples were not covered by a glass cap and held at a distance of 10 cm between the Hg-lamp and the surface of the sample.

After 6 hours, the samples were analyzed for the EDOT-dimer and EDOT-trimer content by ¹H-NMR-spectroscopy in CDCl₃. The intensity of the single proton absorption at 5.42 ppm (δ), corresponding to position 5 in 2,2',3,3',5,7-hexahydro-5,5'-bithieno[3,4-b][1,4]dioxin ("EDOT-dimer") was used to calculate the EDOT-dimer content. The proton resonance coincides with the absorption of the protons at C-atom 5 and 5" in 2,2',2",3,3',3",5,5",7,7"-decahydro-5,5':7,5"-terthieno-[3,4b][1,4]dioxin ("EDOT-trimer"). Because the EDOT-dimer is the main product in the EDOT-dimerization- and trimerization reactions (see EP 1 375 560 B1, H.C. Starck GmbH), and dimer and trimer are not distinguishable by the ¹H-NMR-absorption at 5.42 ppm, the EDOT reaction products are calculated as dimer. 100 ppm was the detection limit.

The following table 1 shows the results with different stabilisers (example 1-15) and demonstrates the inhibition or decrease of the photochemical dimer formation.

| Example/Comparative Example | stabiliser | dimer content [ppm] |
|---|---|---|
| A¹⁾ | unstabilised | 240 |
| 1 | Irganox^{®}1076 | 140 |
| 2 | Tinuvin^{®}765 | n. d.²⁾ |
| 3 | Tinuvin^{®}1130 | n. d. |
| 4 | Tinuvin^{®}770 DF | n. d. |
| 5 | Uvinul^{®}4050 H | n. d. |
| 6 | N-methyl-diethanolamine | n. d. |
| 7 | 2-dimethylaminoethanol | n. d. |
| 8 | triethanolamine | n. d. |
| 9 | Tinuvin^{®}123 | n. d. |
| 10 | Tinuvin^{®}292 | n. d. |
| 11 | 4-tert-butylcatechol | n. d. |
| 12 | 2,2'-methylene-bis-(6-tert-butyl-4-ethyl)phenol | n. d. |
| 13 | propyl gallate | n. d. |
| 14 | 3-tert-butyl-4-hydroxyanisole | n. d. |
| 15 | pyrogallol | n. d. |

| | | |
|---|---|---|
| ¹⁾ Comparative Example ²⁾ n.d. = not detectable | | |

### Examples 16-28:

The procedure from examples 1 - 15 was repeated, with the exception that instead of pure EDOT a pre-deteriorated EDOT (after prolonged storage over several years), containing 1490 ppm EDOT-dimer, was used (calculation from ¹H-NMR, see above). The following table demonstrates the decrease of the additional photochemical dimer formation by different stabilisers:

| Example/Comparative Example | stabiliser | dimer formation [ppm] |
|---|---|---|
| B¹⁾ | unstabilised | 470 |
| 16 | Irganox^{®}1076 | 410 |
| 17 | Tinuvin^{®}765 | 380 |
| 18 | Tinuvin^{®}1130 | 460 |
| 19 | Tinuvin^{®}770 DF | 320 |
| 20 | Uvinul^{®}4050 H | 390 |
| 21 | N-methyl-diethanolamine | 270 |
| 22 | 2-dimethylaminoethanol | 20 |
| 23 | triethanolamine | 270 |
| 24 | Tinuvin^{®}123 | 370 |
| 25 | Tinuvin^{®}292 | 360 |
| 26 | 4-tert-butylcatechol | 270 |
| 27 | 2,2'-methylene-bis-(6-tert-butyl-4-ethyl)phenol | 310 |
| 28 | Irganox^{®} MD 1024 | 270 |

| | | |
|---|---|---|
| ¹⁾ Comparative Example | | |

### Example 29 and Comparative Example C:

In this Example a capacitor is formed using the stabilized monomer composition. As Comparative Example C a monomer has been used that was not stabilized according to the present invention.

### 1. Production of oxidised electrode pellets

Tantalum powder having a specific capacitance of 48,500 µFV/g (VFI-50KD, H.C. Starck GmbH) was mixed with camphor as a binder and then compressed with a side press together with a tantalum anode wire of 0.49 mm diameter into porous electrode bodies (anode pellets) of dimension 4.4 mm × 3.1 mm × 1.0 mm. with a green density of 5.5 g/cm³. The camphor binder was removed by a 90 min heat treatment at 190°C. Then the electrode pellets were sintered for 20 min at 1315°C. The pellets were anodised to 30 V in a phosphoric acid electrolyte of 4300 µS at 85°C. The current density was set to 150 mA per gramm of used tantalum powder. After anodization the pellets were washed at 85°C for 60 min and then dried.

### 2. Formation of polymer solid electrolyte

2.1. A solution was prepared consisting of 1 part by weight of the stabilized monomer composition of Example 4 and 20 parts by weight of a 40 wt.% ethanolic solution of iron(III) p-toluenesulfonate (Clevios C-E, H.C. Starck Clevios GmbH). For Comparative Example C the pure EDOT of Comparative Example A was used.
2.2. The solutions was used to impregnate 9 anode pellets each. The anode pellets were immersed in this solution with an automatic dip coater at a speed of 0.3 mm/s and withdrawn form the solution with a speed of 1 mm/s after a soaking time of 60 s. Then the anodes pellets were exposed for 15 minutes at 25°C to a relative atmospheric humidity of 95%.
2.3 Then process steps 2.1 and 2.2 were repeated on the anode pellets.
2.4. Following this the anodes pellets were heat treated for 30 minutes at 50°C in a drying cabinet. The pellets were then washed for 60 minutes in an aqueous 2% solution of p-toluenesulfonic acid. The anode pellets were re-formed at 30 V for 30 minutes in a aqueous solution of p-toluenesulfonic acid of 4300 µS at 25°C, and then rinsed in distilled water and dried.
2.5 The process steps 2.1 to 2.4 were repeated two times.

### 3. Formation of outer coatings and electrical measurement

868 g deionised water, 330 g of an aqueous polystyrene sulphuric acid solution having an average molecular weight of 70,000 and a solids content of 3.8 % by weight were placed in a 2-1 three-necked flask with stirrer and internal thermometer. The reaction temperature was maintained between 20 and 25°C. 5.1 g 3,4-ethylenedioxythiophene were added while stirring. The solution was stirred for 30 minutes. 0.03 g iron(III) sulphate and 9.5 g sodium persulphate were then added and the solution was stirred for a further 24 hours. Once the reaction has been completed, 100 ml of a strongly acid cation exchanger and 250 ml of a weakly basic anion exchanger were added, for removing inorganic salts, and the solution was stirred for a further two hours. The ion exchanger was filtered out and a poly(3,4-ethylenedioxythiophene)/polystyrene sulphonate dispersion was obtained.

2.51 demineralised water were placed in a 5-1 glass reactor with stirrer and thermometer. 214.2 g p-toluene sulphonic acid monohydrate and 2.25 g iron(III) sulphate heptahydrate were introduced while stirring. Once the entire mixture had dissolved, 85.8 g 3,4-ethylenedioxythiophene were added and stirring was continued for 30 minutes. 192.9 g sodium persulphate were then introduced while stirring, and the mixture was stirred for a further 24 hours at ambient temperature. After the end of the reaction, the PEDT/toluene sulphonate powder was filtered out on a porcelain suction filter, washed with 31 demineralised water and finally dried for 6 hours at 100°C. 89 g of a bluish black PEDT toluene sulphonate powder were obtained.

In a beaker with stirrer, 170 g of the poly(3,4-ethylenedioxythiophene)/polystyrene sulphonate dispersion, 15 g of a water based polyester dispersion, 8 g dimethyl sulphoxide, 1 g 3-glycidoxypropyltrimethoxysilane and 0.4 g of a non-ionic acetylenic-based wetting agent were mixed intensively for one hour. 6 g of the PEDT/toluene sulphonate powder were then dispersed using a bead mill dissolver unit. For this purpose, 300 g of zirconium oxide beads (∅ 1 mm) were added and the mixture was stirred at 7000 rpm for one hour, while being cooled with water. Finally, the ground beads were separated via a 0.8 µm sieve.

The anode pellets which were prepared according to section 1 and 2 of this example were then dipped in the so prepared dispersion and subsequently dried for 10 min at 120°C. The dipping into the dispersion was carried out with an automatic dip coater at a speed of 0.15 mm/s. The anode pellets were withdrawn form the dispersion with a speed of 1 mm/s after a soaking time of 10 s.

Afterwards the pellets were dip-coated with a graphite (mixture of 1 part by weight Electrodag PR406 (Acheson) and 3 parts by weight diethylene glycol butyl ether (Aldrich)) and dried for 30 min at 25°C, 30 min at 50°C and 15 min at 150°C. The pellets were then dip-coated with a silver (Electrodag 503, Acheson) and dried for 15 min at 25°C and 45 min at 150°C.

The capacitance was determined at 120 Hz and the equivalent series resistance (ESR) at 100 kHz using a LCR meter (Agilent 4284A) with a four-point probe.

The average electrical results of the 9 capacitors are given in Table 1. The standard deviation of the average was 0.5 % for capacitance and 1 mΩ for ESR.

| Example/Comparative Example | stabilizer | Capacitance [µF] | ESR [mΩ] |
|---|---|---|---|
| 29 | Tinuvin^{®}770 DF | 89 | 22 |
| C | unstabilized | 89 | 25 |

## Claims

1. Use of a stabiliser selected from the group consisting of a radical scavenger, an amine and an UV/VIS-absorber, for inhibiting the formation of dimeric and trimeric thiophenes in a stabilised monomer composition during storage, the stabilised monomer composition comprising
- at least 90 wt.%, based on the total weight of the stabilised monomer, of 3,4-ethylenedioxythiophene as the monomer,
and
- 0.001 to 10 wt.%, based on the total weight of the stabilised monomer, of the stabiliser,
wherein the stabiliser is completely dissolved in the monomer without the formation of a separate liquid phase or a remaining solid mass and wherein the stabilised monomer composition is contained in a closed container.

2. Use according to claim 1, wherein the stabiliser is a radical scavenger

3. Use according to claim 2, wherein the radical scavenger has a chemical structure comprising at least one structural element having the general formula (III) 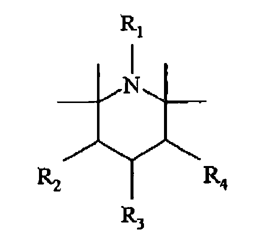 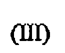 in which
R₁ stands for a hydrogen atom, a hydroxyl group, linear or branched C₁-C₁₈-alkyl residue or a linear or branched C₁-C₁₈alkoxy residue, and
R₂, R₃ and R₄ stand, independently of one another, for a hydrogen atom or an organic residue.

4. Use according to claim 3, wherein the radical scavenger has a chemical structure comprising of the general formula (IV) 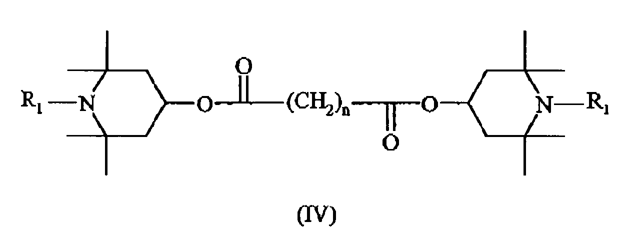 in which R₁ is a residue having the significance stated in claim 3 and n is an integer ranging from 2 to 20.

5. Use according to claim 3, wherein the radical scavenger has a chemical structure comprising of the general formula (V) 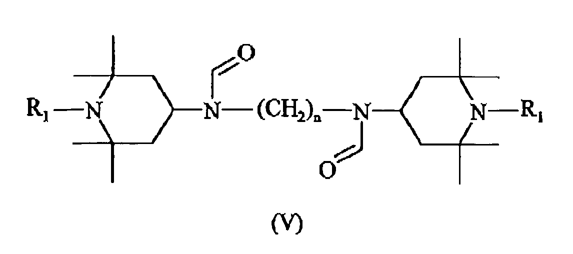 in which R₁ is a residue have the significance stated in claim 3 and n is an integer ranging from 2 to 20.

6. Use according to claim 3, wherein the radical scavenger is a phenol derivative.

7. Use according to claim 6, wherein the radical scavenger has a chemical structure comprising at least one structural element having the general formula (VI) 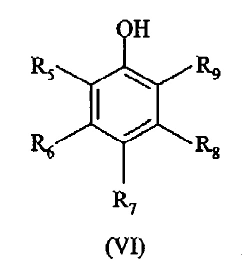 in which R₅, R₆, R₇, R₈ and R₉ stand, independently of one another, for a hydrogen atom, a hydroxyl group or an organic residue, with the proviso that at least one of the residues R₅, R₆, R₇, R₈ and R₉ is a -C(CH₃)₃ residue.

8. Use according to claim 7, wherein one of the residues R₅ and R₇ is a hydroxyl group.

9. Use according to claim 7, wherein the radical scavenger has a chemical structure comprising at least one structural element having the general formula (VII) 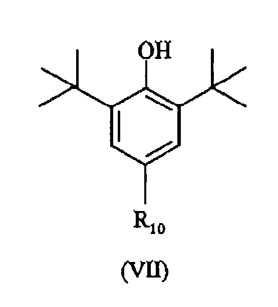 in which R₁₀ stands for a hydrogen atom, a hydroxyl group or an organic residue.

10. Use according to claim 6, wherein the radical scavenger has a chemical structure comprising at least one structural element having the general formula (VIII) 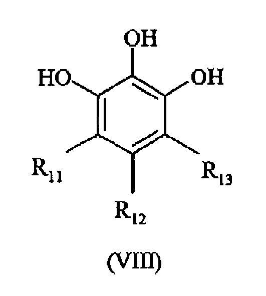 in which R₁₁, R₁₂ and R₁₃ stand, independently of one another, for a hydrogen atom or an organic residue.

11. Use according to claim 1, wherein the stabiliser is an amine.

12. Use according to claim 11, wherein the amine has a chemical structure having the general formula (IX) 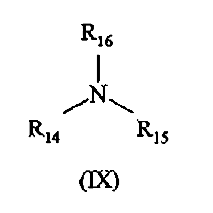 in which R₁₄, R₁₅ and R₁₆ stand, independently of one another, for a hydrogen atom, a C₁-C₂₀-alkyl residue or an C₁-C₅-hydroxyalkyl residue.

13. Use according to claim 1, wherein the stabiliser is an UV/VIS-absorber.

## Patentansprüche

1. Verwendung eines Stabilisators ausgewählt aus der Gruppe bestehend aus einem Radikalfänger, einem Amin und einem UV/VIS-Absorber, zur Inhibierung der Bildung von dimeren und trimeren Thiophenen in einer stabilisierten Monomer-Zusammensetzung während der Lagerung, wobei die stabilisierte Monomer-Zusammensetzung
- mindestens 90 Gew.-%, bezogen auf das Gesamtgewicht des stabilisierten Monomers, 3,4-Ethylendioxythiophen als das Monomer,
und
- 0,001 bis 10 Gew.-%, bezogen auf das Gesamtgewicht des stabilisierten Monomers, des Stabilisators
umfasst, wobei der Stabilisator ohne Bildung einer separaten flüssigen Phase oder einer zurückbleibenden festen Masse vollständig im Monomer gelöst ist und wobei die stabilisierte Monomer-Zusammensetzung in einem verschlossenen Behälter enthalten ist.

2. Verwendung nach Anspruch 1, wobei der Stabilisator ein Radikalfänger ist.

3. Verwendung nach Anspruch 2, wobei der Radikalfänger eine chemische Struktur aufweist, welche mindestens ein strukturelles Element umfasst, welches die allgemeine Formel (III) aufweist 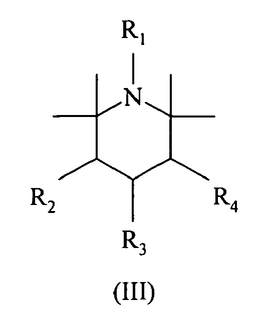 in der
R₁ für ein Wasserstoffatom, eine Hydroxy-Gruppe, einen linearen oder verzweigten C₁-C₁₈-Alkylrest oder einer linearen oder verzweigten C₁-C₁₈-Alkoxy-Rest steht, und
R₂, R₃ und R₄ unabhängig voneinander für ein Wasserstoffatom oder einen organischen Rest stehen.

4. Verwendung nach Anspruch 3, wobei der Radikalfänger eine chemische Struktur aufweist umfassend die allgemeine Formel (IV) 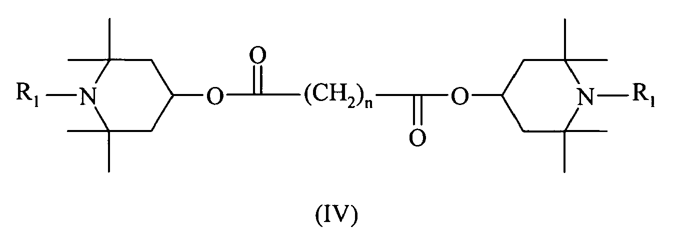 in der R₁ ein Rest mit der in Anspruch 3 angegebenen Bedeutung ist und n eine ganze Zahl in einem Bereich von 2 bis 20 ist.

5. Verwendung nach Anspruch 3, wherein der Radikalfänger eine chemische Struktur aufweist umfassend die allgemeine Formel (V) 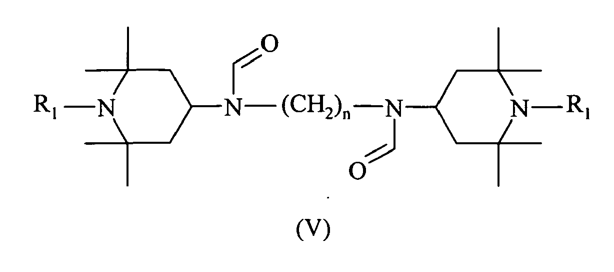 in der R₁ ein Rest mit der in Anspruch 3 angegebenen Bedeutung ist und n eine ganze Zahl in einem Bereich von 2 bis 20 ist.

6. Verwendung nach Anspruch 3, wobei der Radikalfänger ein Phenol-Derivat ist.

7. Verwendung nach Anspruch 6, wobei der Radikalfänger eine chemische Struktur aufweist, welche mindestens ein strukturelles Element umfasst, welches die allgemeine Formel (VI) aufweist 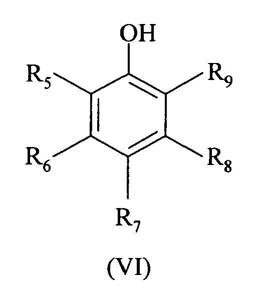 in der R₅, R₆, R₇, R₈ und R₉ unabhängig voneinander für ein Wasserstoffatom, eine Hydroxy-Gruppe oder einen organischen Rest stehen, mit der Maßgabe, das mindestens einer der Reste ein R₅, R₆, R₇, R₈ und R₉ ein Rest -C(CH₃)₃ ist.

8. Verwendung nach Anspruch 7, wobei einer der Reste R₅ und R₇ eine Hydroxy-Gruppe ist.

9. Verwendung nach Anspruch 7, wobei der Radikalfänger eine chemische Struktur aufweist, welche mindestens ein strukturelles Element umfasst, welches die allgemeine Formel (VII) aufweist 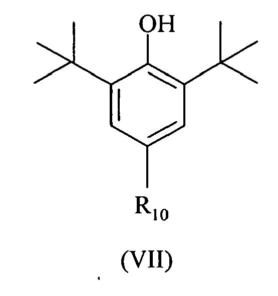 in der R₁₀ für ein Wasserstoffatom, eine Hydroxy-Gruppe oder einen organischen Rest steht.

10. Verwendung nach Anspruch 6, wobei der Radikalfänger eine chemische Struktur aufweist, welche mindestens ein strukturelles Element umfasst, welches die allgemeine Formel (VIII) aufweist 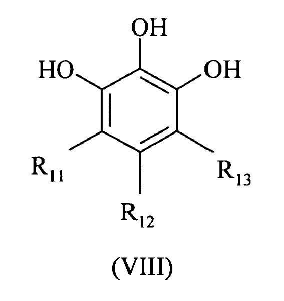 in der R₁₁, R₁₂ und R₁₃ unabhängig voneinander für ein Wasserstoffatom oder einen organischen Rest stehen.

11. Verwendung nach Anspruch 1, wobei der Stabilisator ein Amin ist.

12. Verwendung nach Anspruch 11, wobei das Amin eine chemische Struktur aufweist mit der allgemeinen Formel (IX) 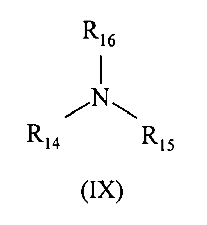 in der R₁₄, R₁₅ und R₁₆ unabhängig voneinander für ein Wasserstoffatom, einen C₁-C₂₀-Alkyl-Rest oder einen C₁-C₅-Hydroxyalkyl-Rest stehen.

13. Verwendung nach Anspruch 1, wobei der Stabilisator ein UV/VIS-Absorber ist.

## Revendications

1. Utilisation d'un stabilisant choisi dans le groupe constitué par un piégeur de radicaux, une amine et un absorbant UV/VIS, pour inhiber la formation de thiophènes dimères et trimères dans une composition monomère stabilisée pendant le stockage, la composition monomère stabilisée comprenant
- au moins 90 % en poids, sur la base du poids total du monomère stabilisé, de 3,4-éthylènedioxythiophène à titre de monomère, et
- 0,001 à 10 % en poids, sur la base du poids total du monomère stabilisé, de stabilisant,
dans laquelle le stabilisant est complètement dissous dans le monomère sans formation d'une phase liquide séparée ou d'une masse solide résiduelle et dans laquelle la composition monomère stabilisée est contenue dans un récipient clos.

2. Utilisation selon la revendication 1, dans laquelle le stabilisant est un piégeur de radicaux.

3. Utilisation selon la revendication 2, dans laquelle le piégeur de radicaux a une structure chimique comprenant au moins un élément structural répondant à la formule générale (III) 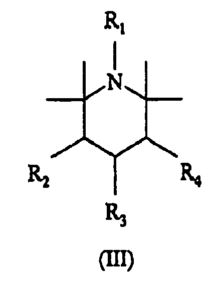 dans laquelle
R₁ représente un atome d'hydrogène, un groupe hydroxyle, un résidu alkyle linéaire ou ramifié C₁-C₁₈ ou un résidu alcoxy linéaire ou ramifié C₁-C₁₈, et
R₂, R₃ et R₄ représentent, indépendamment les uns des autres, un atome d'hydrogène ou un résidu organique.

4. Utilisation selon la revendication 3, dans laquelle le piégeur de radicaux a une structure chimique répondant à la formule générale (IV) 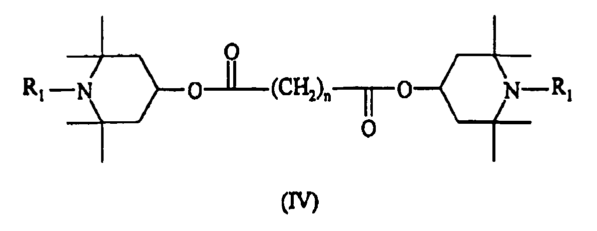 dans laquelle R₁ est un résidu ayant la signification indiquée dans la revendication 3 et n est un entier allant de 2 à 20.

5. Utilisation selon la revendication 3, dans laquelle le piégeur de radicaux a une structure chimique répondant à la formule générale (V) 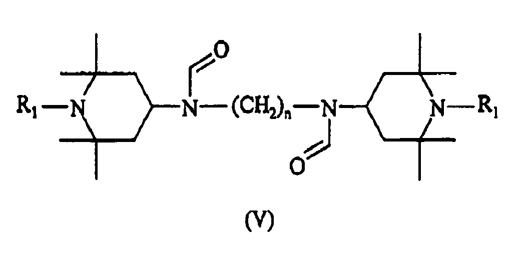 dans laquelle R₁ est un résidu ayant la signification indiquée dans la revendication 3 et n est un entier allant de 2 à 20.

6. Utilisation selon la revendication 3, dans laquelle le piégeur de radicaux est un dérivé de phénol.

7. Utilisation selon la revendication 6, dans laquelle le piégeur de radicaux a une structure chimique comprenant au moins un élément structural répondant à la formule générale (VI) 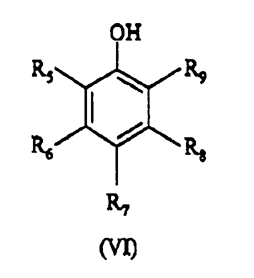 dans laquelle R₅, R₆, R₇, R₈ et R₉ représentent, indépendamment les uns des autres, un atome d'hydrogène, un groupe hydroxyle ou un résidu organique, à condition qu'au moins un des résidus R₅, R₆, R₇, R₈ et R₉ soit un résidu -C(CH₃)₃.

8. Utilisation selon la revendication 7, dans laquelle un des résidus R₅ et R₇ est un groupe hydroxyle.

9. Utilisation selon la revendication 7, dans laquelle le piégeur de radicaux a une structure chimique comprenant au moins un élément structural répondant à la formule générale (VII) 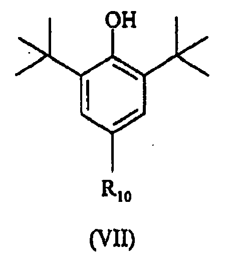 dans laquelle R₁₀ représente un atome d'hydrogène, un groupe hydroxyle ou un résidu organique.

10. Utilisation selon la revendication 6, dans laquelle le piégeur de radicaux a une structure chimique comprenant au moins un élément structural répondant à la formule générale (VIII) 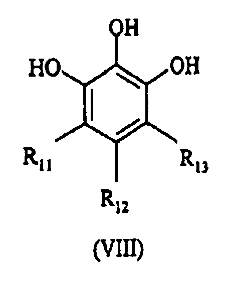 dans laquelle R₁₁, R₁₂ et R₁₃ représentent, indépendamment les uns des autres, un atome d'hydrogène ou un résidu organique.

11. Utilisation selon la revendication 1, dans laquelle le stabilisant est une amine.

12. Utilisation selon la revendication 11, dans laquelle l'amine a une structure chimique répondant à la formule générale (IX) 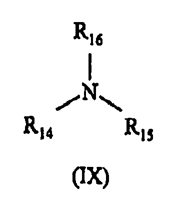 dans laquelle R₁₄, R₁₅ et R₁₆ représentent, indépendamment les uns des autres, un atome d'hydrogène, un résidu alkyle C₁-C₂₀ ou un résidu hydroxyalkyle C₁-C₅.

13. Utilisation selon la revendication 1, dans laquelle le stabilisant est un absorbant UV/VIS.
